# EUROPEAN PATENT APPLICATION

(11) **EP 1 964 541 A1**
(43) Date of publication of application: **03.09.2008**
(21) Application number: 07290268.7
(22) Date of filing: 02.03.2007
(51) Int. Cl.: A61K 8/33, A61K 8/34, A61K 8/35, A61K 8/37

(54) **Preservative compositions**

(71) Applicant: Takasago International Corporation, Tokyo-to 144-8721 (JP)
(72) Inventor: Warr, Jonathan, 75017 Paris (FR); Fraser, Stuart, Cheshire - CH64 4DH (GB); Kl Kang, Raphael, Englewood, NJ 07631 (US); Gouault, Olivier, 92320 Chatillon (FR)
(74) Representative: Gillard, Marie-Louise

(57) **Abstract**

The present invention relates to a preservative composition comprising:
a mixture of 2-10 well characterized fragrance raw materials with a cosmetic function, of which at least 2 are selected from: allyl caproate, benzyl acetate, benzaldehyde, dihydroisojasmonate, ethyl phenethylacetal, ethyl cinnamate, ethyl methyl phenyl glycidate, ethyl vanillin, 2-heptylcyclopentanone, geranyl acetate, heliotropine, cis hex-3-en-1-ol, ethylene brassylate, nonalactone gamma, camphylcyclohexanol, undecalactone gamma, 2-t-butylcyclohexylacetate, pentyl salicylate, 2-phenylethanol and 2-phenylethyl acetate
and represent at least 20% by weight of the said mixture, and at least one preservative and
a sequestrant.

Application: perfumed cosmetic, personal care and liquid detergent compositions containing a preservative

## Description

### Field of the Invention

The present invention relates to cosmetic, personal care and liquid detergent compositions and more particularly, to perfumed cosmetic, personal care and liquid detergent compositions containing a preservative the beneficial effect of which is enhanced by the selection of specific well characterized fragrance ingredients.

### Background of the Invention

Most cosmetic, personal care and liquid detergent compositions e.g. shampoos, bath foams, skin creams, washing-up liquids and the like are susceptible to microbial spoilage. Such spoilage cannot only compromise the performance of the product but also, in extreme cases, present a health hazard to users of the product. Of course, manufacturers of such products take stringent precautions to prevent microbial contamination of their products during manufacture and distribution. Once the product package has been opened however, the contents may be open to inadvertent contamination by the user or e.g. through airborne spores.

It is customary, therefore, to add a preservative to such products at the time of manufacturing in order to protect the product against microbial contamination in the long term. A wide variety of such preservatives are known and used. The precise choice of type and level of the preservative is usually made by the formulator based upon a number of factors including, for example, the microbiological requirements of the product, cost, the pH of the product, compatibility with the other formulation ingredients and regulatory restrictions. A guide to the factors used in preservative selection and testing can be found in 'Cosmetic and Drug Preservation, Vol.1, Principles and Practice', published by Marcel Dekker Inc.

It is widely known that many conventional perfume ingredients, essential oils as well as single perfume chemicals, have been tested for antimicrobial properties against many different microorganisms with differing results and conclusions. Thus, S. G. Dean and G. Ritchie, Int. Journal of Food Microbiology. 5 (1987), pp 165-180, examined 50 essential oils for their antibacterial properties against 25 genera of bacteria using an agar diffusion technique and determining zones of inhibition. They concluded that gram-positive as well as gram-negative bacteria were susceptible to (some of the) essential oils.

J. Blakeway, Soap Perfumery & Cosmetics. April 1986, 201-203 determined the antimicrobial activity of 50 essential oils against 22 microorganisms (bacteria, yeasts and fungi) also measuring zones of inhibition. His conclusion was that essential oils have generally little activity against gram-negative bacteria. Angelica for instance, which was found to be active against all 25 bacteria including *Escherichia coli, Serratia marcescens* and *Pseudomonas aeruginosa,* by Dean and Ritchie, is described as inactive against these bacteria by Blakeway.

J. A. Morris et al, J. Am. Oil Chem. Soc. 56 (1979), 595-603 tested 521 perfume ingredients, including essential oils and single perfume chemicals, against *Staphylococcus aureus, Escherichia coli* and *Candida albicans* and 212 of these also against a *Corynebacterium sp.* They measured zones of inhibition and the positive ingredients were subjected to a minimum inhibitory concentration test in a liquid culture. 44% of the 521 were inhibitory against at least 1 microorganism and 12% against all three.

H. P. Munzing and H. Schels, J. Soc. Cosmetic Chemists 23 (1972), 841-852 measured the activity of 37 perfume ingredients of natural and synthetic origin in a liquid culture against *Staphylococcus aureus, Escherichia coli, Proteus vulgaris* and *Pseudomonas aeruginosa.* They again found that the gram positive *S*. *aureus* was more affected than the gram-negative bacteria. Furthermore they found random activity of the separate ingredients against the different microorganisms, which means that it seems to be impossible to predict the activity of a specific perfume ingredient against one microorganism on the basis of its activity against another microorganism. From their results they were able to prepare simple perfume compositions which in liquid culture inhibited growth of three bacteria, but not of *Ps. aeruginosa.* One composition was moderately active against these three bacteria when added in 0.2% to an O/W emulsion.

H. Isacoff in "The Chemistry and Manufacture of Cosmetics", Chapter 8, (Ed. M. G. de Navarre), Vol. III, 2nd Edition, Orlando 1975, reviewed the existing literature on preservation with essential oils and perfume compounds and based on this information prepared two perfume compositions which were tested as preservatives in O/W emulsions against *Staphylococcus aureus, Pseudomonas aeruginosa, Bacillus subtilis, Aspergillus niger* and *Candida albicans,* with mixed success. A further review of the literature was prepared by J. J. Kabara, "Aroma Preservatives" in "Cosmet. Sci. Technol. Ser, Vol 1 (Cosmet. Drug Preserv. 1984)." 237-273. He did not cite any further examples of actual perfume compositions which were tested, either as such or after application in a product, for their antimicrobial activity. W. Sturm, Soap Perfumery & Cosmetics 1979, 475-478 described the repeated use of well known but unspecified perfume ingredients to inhibit or reduce the growth of equally unspecified skin bacteria. Thus while there is considerable evidence that essential oils and perfumery ingredients have antibacterial effects, it is less clear how effective these materials are as product preservatives across both the wide range of household and personal care product formulations available and the wide range of contaminant organisms.

In EP 0 144 417 B1 the use of mixtures of cinnamaldehyde and parabens as preservatives is described. EP 0 570 794 A2 claims preservative benefits from the combination of antimicrobial perfumery ingredients and a select group of acids. US Patent 5,306,707 teaches that antimicrobially active perfume ingredients, having a substituted benzene group as part of the chemical structure, can be used together with low levels of conventional preservatives, to preserve aqueous based, surfactant containing products. In the latter two documents, test methods are described to determine whether a particular combination of fragrance ingredients has any preservative effect in specific product formulations for specified target organisms. It is apparent, from the frequency of reports that some compounds are more highly effective than others for specific organisms, while some materials have a broader spectrum of activity than others. These more active compounds are likely to be more frequently employed and used at higher levels in formulating preservative fragrances than would normally be the case.

Despite the substantial published literature there is not sufficient data describing the antimicrobial activity of the wide range of fragrance materials against the various microorganisms which could spoil a product tested under conditions which simulate cosmetic, toiletry, personal care and household and laundry products.

Thus, one may conclude that while some essential oils and fragrance molecules undoubtedly have antimicrobial effects it is still not possible to predict whether particular combinations of fragrance materials will be effective as preservatives in specific product formulations.

An effective preservative is one which prevents the multiplication of any bacteria, mould, yeast or fungi i.e. possessing either or both biocidal and biostatic effects, over a product storage lifetime and while in use. Many factors influence product preservation among which are: the specific product composition, its pH, hygiene during manufacture; its manner of use, expected life in use, and the frequency and severity of organism inoculation in use which is influenced by the type of packaging and storage temperature.

It has now be found that products can be preserved using lower than normal levels of traditional chemical preservatives by including in the formulation well characterized fragrance ingredients having a cosmetic function and optionally sequestrants which together enhance the preservation of the composition as a whole.

Without wishing to be bound by theory it is believed that a combination of ingredients may work together to inhibit several different biochemical processes leading to improved product preservation across a broader range of microorganisms than a single preservative can usually achieve. Fragrance materials having a cosmetic function are especially relevant to the present invention since they can and often are used at higher levels than conventional fragrance materials to fulfill another purpose within a cosmetic product.

Fragrances used previously as preservatives either rely on dosing higher than normal perfume levels which may not be economic, or which may leave an overpowering fragrance or high levels of particularly effective compounds may be dosed dominating the fragrance note and restricting the creativity of the perfumer.

Surprisingly it has also been discovered that pleasant and customer desirable fragrances can be created from combinations of a small number of fragrance materials having a cosmetic function, in contrast to the majority of commercial product fragrances which contain large numbers of ingredients. Thus it is possible to satisfy the requirements of adding sufficient amounts of the materials to improve product preservation whilst still creating attractive fragrances.

### Summary of the Invention

This invention concerns a preservative composition which may be used in cosmetics, toiletries, personal cleansing and care products and household cleaning and laundry products containing at least 15% water.

The preservative composition of the present invention comprises:
a) 5-80% by weight of a mixture of 2-10 well characterized fragrance raw materials with a cosmetic function, of which at least 2 are selected from:
   allyl caproate, benzyl acetate, benzaldehyde, dihydroisojasmonate, ethyl phenethylacetal, ethyl cinnamate, ethyl methyl phenyl glycidate, ethyl vanillin, 2-heptylcyclopentanone, geranyl acetate, heliotropine, cis hex-3-en-1-ol, ethylene brassylate, nonalactone gamma, camphylcyclohexanol, undecalactone gamma, 2-t-butylcyclohexylacetate, pentyl salicylate, 2-phenylethanol and 2-phenylethylacetate,
   and represent at least 20% by weight of the said mixture, and
b) 0-80% by weight of one or more of the following preservatives:
   parahydroxy benzoic acid and salts thereof, (C₁-C₄) alkyl parahydroxy benzoates, sorbic acid and salts thereof, ortho hydroxybenzoic acid and salts thereof, benzoic acid and salts thereof, (C₁-C₄) alkyl benzoates, propionic acid and salts thereof, dehydroacetic acid and salts thereof, formic acid and salts thereof, undec-10-enoic acid and salts thereof, and
either c) 10-80% by weight of one or more of the following preservatives:
   formaldehyde, paraformaldehyde, biphenyl-2-ol (ortho phenylphenol), 4,4-dimethyloxazolidine, 5-bromo-5-nitro-1,3 dioxane, 2-bromo-2-nitropropane-1,3-diol, 2,4 dichlorobenzyl alcohol, 5-chloro-2-(2,4-dichlorophenoxy)-phenol, 4-chloro-3,5-xylenol, 3,3'-bis (1-hydroxymethyl-2,5-dioxoimidazolidinyl-4-yl)-1,1'-methylenediurea (imidazolidinyl urea), poly(1-hexamethylene biguanidine hydrochloride), 2-phenoxyethanol, hexamethylenetetramine, benzyl alcohol, 1,3 Bis (hydroxymethyl)-5,5-dimethylimidazolidine -2,4-dione, 5-chloro-2-methylisothiazol-3(2H)-one, 2-methyl-isothiazol-3(2H)-one, benzisothiazolinone, 2-benzyl-4-chlorophenol, Chlorhexidine and its digluconate, diacetate, and dihydrochloride salts, 1-phenoxy-propan-2-ol, cetyl pyridinium bromide and chloride, N-(hydroxymethyl)-N-dihydroxymethyl-1,3-dioxo-2,5-imidazolinidyl-4)-N'-hydroxymethyl urea, sodium hydroxymethylglycinate, benzethonium chloride, Benzalkonium chloride, bromide and saccharinate, 3-iodopropynylbutylcarbamate, benzisothiazolinone, triacetin, cis-1-(3-chloroallyl-3,5,7-triaza-1-azoniaadamantane chloride;
and/or d) 1-90% by weight of one or more of sequestrants selected among amino carboxylates, amino phosphonates, polyfunctionally-substituted aromatic chelating agents and polycarboxylic acids and salts thereof;
wherein the preservative composition optionally contains e) 0.1-50% by weight of one or more natural extracts, and the sum of a)-e) must equal 100% by weight.

### Detailed Description of the Invention

The preservative composition of the invention comprises a combination of ingredients, which, when used in a cosmetic, toiletry, personal care, household or laundry product, improves the preservation of that product to microbial spoilage compared with the preservative alone. In the following description, the cosmetic, toiletry, personal care, household or laundry products are named "final products"

These ingredients may be premixed before adding to the final product but it is not a requirement of the invention to do so. Individual ingredients of the composition may be added to a product at convenient times during the manufacturing process.

Improved preservative effect may be defined either as superior biocidal effect i.e. a reduction in the number of colonies of microbes surviving after a set time exposed to the composition in a model product composition in the preservation test described later. Alternatively improved preservation may be defined as equivalent efficacy but with reduced dosage of any conventional preservatives within the composition.

### Well Characterized Fragrance Materials

In the context of this specification a "well characterized fragrance material", which term is synonymous with a "well characterized perfume material" or "well characterized perfume or fragrance ingredient" is an essential part of the invention.

For the purpose of the invention, the expression "well characterized fragrance material having a cosmetic function" means a fragrance having an INCI name (defined by the International Nomenclature of Cosmetic Ingredients), which has purity greater than 90% by weight as defined below and has a cosmetic function.

In commercial fragrance ingredients it is not possible to achieve total purity and even among different batches of the same material from the same supplier it is possible for the levels of minor compounds to vary. Isomerisation, rearrangement, hydrogenation and dehydrogenation are examples of chemical changes, which can occur during manufacture and materials of this type structurally related to the major compounds are not considered to be impurities. Materials of this type which are structural, geometric or optical isomers or which differ by no more than 2 mass units from the major compound, specifically when one single bond is replaced by a double bond or vice versa having the same carbon skeleton and functional groups other than saturation/unsaturation are not considered to be impurities. For the purpose of the invention, well characterized fragrance ingredients of the current invention may contain up to 10% by weight of other materials and even more preferably they contain less than 1% by weight of other materials.

Well characterized fragrance ingredients may result from chemical synthesis using available commercial chemicals, or by purification of natural materials, or by chemical reactions on a naturally derived raw material or from microbial biosynthesis. Well characterized fragrance ingredients may well comprise mixtures. These mixtures can include different chemical species, such as starting materials together with reaction products and/or mixtures of isomers, i.e. compounds having the same chemical formula and therefore the same molecular weight. Isomers may be structural which means that compounds have the same chemical formula and molecular weight but the atoms are joined together in a different order. Thus, n-pentanol and isopentanol are structural isomers and for the purposes of this specification these are considered as the same ingredient. Stereoisomers have atoms joined in the same order but with a different spatial arrangement and can be subdivided into geometric isomers or optical isomers. Thus alpha amyl cinnamic aldehyde exists as cis-and trans-stereoisomers of the double bond, which are considered here as a single ingredient. Optical isomers are molecules which cannot be superimposed on their mirror images. While most naturally derived compounds are single isomers, many chemical reactions generate mixtures of optical isomers and such mixtures are considered single chemical species for the purposes of this specification.

Perfume ingredients are described more fully in S. Arctander, Perfume Flavors and Chemicals. Vol. I and II, Montclair, N.J., and the Merck Index, 8th Edition, Merck & Co., Inc. Rahway, N.J., both are incorporated herein by reference. However it is well known that some fragrance materials can be harmful, irritating the skin or sensitive areas such as the eyes or cause an allergic reaction in sensitized subjects. Perfume manufacturers are careful in their use of fragrance ingredients to comply with guidelines laid down by industry bodies such as The International Fragrance Research Association (IFRA) and relevant national legislation.

A category of fragrance materials which are particularly safe across a wide variety of product applications and dosage levels are those which have a cosmetic function. These ingredients are well established either through widespread use over along period of time or more recent introductions which are supported by extensive safety testing.

For the purpose of the invention, well characterized fragrance materials having a cosmetic function can be defined as those fragrance materials that are used for other purposes within a cosmetic, toiletry or personal care formulation as described in Commission Decision of European Communities 2006/257/EU of February 9, 2006 amending Decision 96/335/EC, which is subject to amendments and corrections, but excluding those fragrance materials which have a preservative or antimicrobial cosmetic function such as benzyl alcohol and phenyl salicylate.

EU Commission Decision 96/335/EC established an inventory and a common nomenclature of ingredients employed in cosmetic products (INCI) and was amended by Decision 2006/257/EC.

The Cosmetic, Toiletry and Fragrance Association periodically publishes up-dated list of INCI materials. The most recent edition is International Cosmetic Ingredient Dictionary and Handbook, 11th Ed. (2006).

In the Commission Decision of European Communities 2006/257/EU, the cosmetic functions of ingredients are listed and defined in its annex 1, section 1, which is incorporated herein by reference. The well characterized fragrance ingredients of the invention are also listed in annex 1, section 1, and have an International Nomenclature of Cosmetic Ingredients (INCI) name. Well characterized fragrance ingredients having the following cosmetic functions are preferred for the preservative compositions of the invention: stabilizing, emollient, viscosity controlling, antifoaming, solvent, hydrotrope, skin conditioning, emulsifying, toning, denaturing, refreshing, UV filter, UV absorbing, humectant, antistatic, antioxidant, deodorant, hair drying, hair conditioning, plasticizer, soothing and masking.

Particularly preferred fragrance ingredients are those which have the following cosmetic functions: stabilizing, emollient, viscosity controlling, antifoaming, solvent, hydrotrope, skin conditioning, emulsifying, soothing toning, refreshing, UV filter, UV absorbing, humectant, antistatic, antioxidant and deodorant.

Without wishing to be limited, the well characterized perfume ingredients having a cosmetic function of the inventive composition will preferably have molecular weights of less than 325 atomic mass units, preferably less than 300 atomic mass units and more preferably less than 275 atomic mass units to be sufficiently volatile to be perceived. Furthermore the perfume compounds will have preferably molecular weights greater than 70 atomic mass units, preferably greater than 100 atomic mass units as lower masses may be too volatile or too water soluble to function as perfumes. Well characterized perfume ingredients can be found in S. Arctander, Perfume Flavors and Chemicals. Vols. I and II, Montclair, N.J.

Well characterized perfume ingredients of the preservative compositions will not contain strongly ionizing functional groups such as sulphonates, sulphates, or quaternary ammonium ions, nor will they contain any halogen atoms.

Table 1 below lists a number of preferred well characterized fragrance materials which have cosmetic functions and which are capable, in admixture with other ingredients, of improving the preservative effect of an invention composition as hereinabove defined.

A particularly preferred list of well characterized fragrance ingredients are those included in table 2 below,

**TABLE 1: Preferred well characterized fragrance materials having a cosmetic function**

| **Compound** | **CAS no** | **INCI name** | **Other material (%) by weight** | **Cosmetic function** |
|---|---|---|---|---|
| Acetanilide | 103-84-4 | Acetanilid | Less than 1% | Stabilising |
| Pentyl salicylate | 2050-08-0 | Amyl salicylate | Less than 1% | Skin conditioner |
| Hexanol | 111-27-3 | Hexyl alcohol | Less than 1% | Antifoaming/solvent/ hydrotrope |
| Octanol | 111-87-5 | Caprylic alcohol | Less than 1% | Viscosity controlling |
| Aldehyde C-14 (undecalactone gamma) | 104-67-6 | Gamma-undecalactone | Less than 1% | Solvent/masking |
| Aldehyde C-16 (Ethyl Methyl Phenyl Glycidate) | 77-83-8 | Ethyl methylphenyl Glycidate | Less than 1% | Viscosity controlling |
| Aldehyde C-18 (nonalactone, gamma) | 104-61-0 | Gamma-nonalactone | Less than 1% | Solvent |
| Allyl caproate | 123-68-2 | Allyl caproate | Less than 1% | Emollient |
| Amyl acetate | 628-63-7 | Amyl acetate | Less than 1% | Solvent |
| Amyl benzoate | 2049-96-9 | Amyl benzoate | Less than 1% | Solvent/masking |
| Amyl cinnamic aldehyde | 122-40-7 | Amyl cinnamal | Less than 1% | Masking |
| Benzyl acetate | 140-11-4 | Benzyl acetate | Less than 1% | Solvent |
| Benzaldehyde | 100-52-7 | Benzaldehyde | Less than 1% | Solvent |
| Benzyl salicylate | 118-58-1 | Benzyl salicylate | Less than 1% | UV absorber |
| Cinnamic alcohol | 104-54-1 | Cinnamyl alcohol | Less than 1% | Masking |
| Citral | 5392-40-5 | Citral | Less than 1% | Masking |
| Citronellol | 106-22-9 | Citronellol | Less than 5% | Masking |
| Citronellyl acetate | 150-84-5 | Citronellyl acetate | Less than 5% | Masking |
| Costaulon^{®} | 67770-79-0 | Ethyl butylvalerolactone | Less than 1% | Tonic |
| Coumarin | 91-64-5 | Coumarin | Less than 1% | Masking |
| Cyclamen aldehyde | 103-95-7 | Cyclamen aldehyde | Less than 1% | Masking |
| Cyclopentadecanolide | 106-02-5 | Pentadecalactone | Less than 1% | Masking |
| Decalactone | 705-86-2 | Delta-decalactone | Less than 1% | Masking |
| Decanal | 112-31-2 | Decanal | Less than 5% | Masking |
| Dihydro isojasmonate | 37172-53-5 | Dihydrojasmonate | Less than 1% | Tonic |
| Dipropylene glycol | 25265-71-8 | Dipropylene glycol | Less than 10% | Solvent |
| Efetaal^{®} | 2556-10-7 | Ethyl phenethylacetal | Less than 1% | Solvent/tonic |
| Ethyl acetate | 141-78-6 | Ethyl acetate | Less than 1% | Solvent |
| Ethyl cinnamate | 103-36-6 | Ethyl cinnamate | Less than 1% | UV absorber |
| Ethyl laurate | 106-33-2 | Ethyl laurate | Less than 1% | Emollient |
| Ethyl pelargonate | 123-29-5 | Ethyl pelargonate | Less than 1% | Emollient |
| Ethyl vanillin | 121-32-4 | Ethyl vanillin | Less than 1% | Soothing/masking |
| Eugenol | 97-53-0 | Eugenol | Less than 1% | Denaturant/tonic |
| Fleuramone^{®} | 137-03-1 | 2-Heptylcyclopentanone | Less than 1% | Solvent |
| Geraniol | 106-24-1 | Geraniol | Less than 1% | Tonic |
| Geranyl acetate | 105-87-3 | Geranyl acetate | Less than 5% | Tonic |
| Heliotropine | 120-57-0 | Heliotropine | Less than 1% | Masking/ skin conditioning |
| Cis hex-3-en-1-ol, | 928-96-1 | 3-Hexenol | Less than 1% | Solvent/ humectant/ viscosity controlling |
| Hyacinth body^{®} | 2556-10-7 | Ethyl phenethylacetal | Less than 1% | Solvent/tonic |
| Hydroxycitronellal | 107-75-5 | Hydroxycitronellal | Less than 1% | Masking |
| Iso amyl alcohol | 123-51-3 | Iso amyl alcohol | Less than 5% | Solvent |
| Iso amyl acetate | 123-92-2 | Isoamyl acetate | Less than 1% | Solvent |
| Iso butyl acetate | 110-19-0 | Isobutyl acetate | Less than 1% | Solvent |
| Iso eugenol | 97-54-1 | Isoeugenol | Less than 1% | Masking |
| Isopropyl myristate | 11-21-0 | Isopropyl myristate | Less than 5% | Binding/emollient/solve nt/skin conditioner |
| Linalool | 78-70-6 | Linalool | Less than 5% | Deodorant |
| Linalyl acetate | 115-95-7 | Linalyl acetate | Less than 5% | Masking |
| Menthyl acetate | 89-48-5 | Menthyl acetate | Less than 1% | Refreshing/masking |
| Methyl dihydrojasmonate | 2630-39-9 | Methyldihydrojasmonate | Less than 1% | Masking |
| Methyl salicylate | 119-36-8 | Methyl salicylate | Less than 1% | Denaturing/soothing |
| Ethylene brassylate | 105-95-3 | Ethylene brassylate | Less than 10% | Tonic/masking |
| Phenoxanol | 55066-48-3 | Phenylisohexanol | Less than 1% | Masking |
| Propyl acetate | 109-60-4 | Propyl acetate | Less than 1% | Solvent |
| Santalex T^{®} | 68877-29-2 | Camphylcyclohexanol | Less than 10% | Solvent |
| Triethyl citrate | 77-93-0 | Triethyl citrate | Less than 1% | Antioxidant/ deodorant/solvent |
| Vanillin | 121-33-5 | Vanillin | Less than 1% | Masking |
| Verdox^{®} | 88-41-5 | 2-t-Butylcyclohexylacetate | Less than 1% | Solvent |
| 2-Phenyl ethanol | 60-12-8 | Phenylethanol | Less than 5% | Deodorant |
| 2-Phenylethyl acetate | 103-45-7 | Phenyl ethyl acetate | Less than 5% | Deodorant |
| Cinnamic aldehyde | 104-55-2 | Cinnamal | Less than 10% | Denaturant |
| Camphor | 464-99-3 | Camphor | Less than 10% | Denaturant/ plasticizer |
| Thymol | 89-93-8 | Thymol | Less than 1% | Hair drying/denaturant/ masking |
| Menthol | 89-78-1 | Menthol | Less than 5% | Denaturant/soothing/ Refreshing/masking |
| Anethol | 104-46-1 | Anethol | Less than 1% | Denaturant |
| Benzophenone | 119-61-9 | Benzophenone | Less than 1% | UV absorber |
| Benzyl cinnamate | 103-41-3 | Benzyl cinnamate | Less than 1% | Masking |
| Butyl acetate | 123-86-4 | Butyl acetate | Less than 1% | Solvent |
| Carvone | 6485-40-1 & 99-49-0 | Carvone | Less than 1% | Masking |
| Cinnamyl acetate | 103-54-8 | Cinnamyl acetate | Less than 1% | Masking |
| Citronellal | 106-23-0 | Citronellal | Less than 5% | Masking |
| Decenal | 65405-70-1 | Decenal | Less than 5% | Masking |
| Eucalyptol | 470-82-6 | Eucalyptol | Less than 1% | Denaturant/tonic |
| Methyl anthranylate | 134-20-3 | Methyl anthranylate | Less than 1% | Masking |
| Paracymene | 99-87-6 | P-cymene | Less than 5% | Masking |
| Farnesol | 4602-84-0 | Farnesol | Less than 5% | Soothing/solvent/deodor ant |
| Terpineol | 98-55-5 | Terpineol | Less than 10% | Denaturant/solvent |
| Hinokitiol | 499-44-5 | Hinokitiol | Less than 1% | Antistatic/hair conditioning |

**Table 2: Particularly preferred list of well characterized fragrance materials**

| **Compound** | **CAS no** | **INCI name** | **Cosmetic function** |
|---|---|---|---|
| Aldehyde C-14 (undecalactone gamma) | 104-67-6 | Gamma-undecalactone | Solvent |
| Aldehyde C-16 (Ethyl Methyl Phenyl Glycidate) | 77-83-8 | Ethyl methylphenyl glycidate | Viscosity controlling |
| Aldehyde C-18 (nonalactone, gamma) | 104-61-0 | Gamma-nonalactone | Solvent |
| Allyl caproate | 123-68-2 | Allyl caproate | Emollient |
| Pentyl salicylate | 2050-08-0 | Amyl salicylate | Skin conditioner |
| Benzyl acetate | 140-11-4 | Benzyl acetate | Solvent |
| Benzaldehyde | 100-52-7 | Benzaldehyde | Solvent |
| Dihydroisojasmonate | 37172-53-5 | Dihydrojasmonate | Tonic |
| Efetaal^{®} | 2556-10-7 | Ethyl phenethylacetal | Solvent/tonic |
| Ethyl cinnamate | 103-36-6 | Ethyl cinnamate | UV absorber |
| Ethyl vanillin | 121-32-4 | Ethyl vanillin | Soothing |
| Fleuramone^{®} | 137-03-1 | 2-Heptylcyclopentanone | Solvent |
| Geranyl acetate | 105-87-3 | Geranyl acetate | Tonic |
| Heliotropine | 120-57-0 | Heliotropine | Skin conditioning |
| Cis hex-3-en-1-ol | 928-96-1 | 3-hexenol | Solvent/humectant/viscosity controlling |
| Ethylene brassylate | 105-95-3 | Ethylene brassylate | Tonic |
| Santalex T^{®} | 68877-29-2 | Camphylcyclohexanol | Solvent |
| Verdox^{®} | 88-41-5 | 2-t-Butylcyclohexylacetate | Solvent |
| 2-Phenyl ethanol | 60-12-8 | Phenylethanol | Deodorant |
| 2-Phenylethyl acetate | 103-45-7 | Phenyl ethyl acetate | Deodorant |

Fragrances for consumer products usually require a large number of fragrance ingredients to perform satisfactorily. Typically commercial fragrances may contain from 20 to 200 individual ingredients. While it is known to use a single material as a product fragrance the result is usually inferior to a fully formulated fragrance. As the number of fragrance ingredients increases so does the creative scope, however to be effective as an antimicrobial agent a minimum quantity of any active fragrance ingredient will be required. Thus these opposing requirements mean that a balance has to be achieved. So for fragrance quality and breadth of antimicrobial effectiveness at least two or more well characterized fragrance ingredients having a cosmetic function are required, preferably more than 3 ingredients and especially preferably more than 4 ingredients. To avoid overdilution of the effective well characterized fragrance ingredients having a cosmetic function it is desirable if the total number of well characterized fragrance ingredients is 10 or less, preferably 9 ingredients or less, more preferably 8 ingredients or less and especially preferably 7 ingredients or less.
In order to ensure that sufficient well characterized fragrance ingredients are present in the preservative composition, the fragrance ingredients must comprise between 5% by weight and 80% by weight of the preservative composition, preferably between 10% by weight and 70% by weight and more preferably between 15% by weight and 60% by weight. Within the fragrance composition at least 20% by weight, preferably at least 30% by weight more preferably 40% by weight and especially preferably 50% by weight of the fragrance must comprise 2-10 materials chosen from among: allyl caproate, benzyl acetate, benzaldehyde, dihydroisojasmonate, ethyl phenethylacetal, ethyl cinnamate, ethyl methyl phenyl glycidate, ethyl vanillin, 2-heptylcyclopentanone, geranyl acetate, heliotropine, cis hex-3-en-1-ol, ethylene brassylate, nonalactone gamma, camphylcyclohexanol, undecalactone gamma, 2-t-butylcyclohexylacetate, pentyl salicylate, 2-phenylethanol and 2-phenylethyl acetate

Particularly preferred fragrance materials are allyl caproate, benzyl acetate, benzaldehyde, dihydroisojasmonate, ethyl cinnamate, ethyl methyl phenyl glycidate, ethyl vanillin, geranyl acetate, heliotropine, cis hex-3-en-1-ol, ethylene brassylate, nonalactone gamma, camphylcyclohexanol, undecalactone gamma, pentyl salicylate, 2-t-butylcyclohexylacetate

### Exclusions and Limitations

The invention specifies precise numbers of well characterized fragrance ingredients and some fragrance ingredients may not be chemically pure compounds e.g. natural extracts and essential oils which may be variable in composition from different sources, methods of extraction, even seasonal variations. Hence for the purposes of the present invention essential oils and natural extracts are excluded from the definition well characterized fragrance ingredients although it is recognized that some essential oils have antimicrobial benefits. It is also apparent that essential oils and various extracts are widely used in toiletry and personal care products as fragrance ingredients or for the many other benefits they provide; thus, essential oils are optional ingredients of the invention composition but are excluded from any antimicrobial testing so do not contribute to the antimicrobial effect of the invention composition.

Well characterized fragrance materials with a cosmetic function as preservative or antimicrobial for the purposes of this invention are considered as preservatives.

It has also been found that diethyl phthalate diminishes the benefit of adding other well characterized fragrance ingredients and the effect is greater than that which is accounted for by a simple dilution effect. See test examples 12 and 13. Thus diethyl phthalate promotes microbial growth, perhaps by inhibiting the other antimicrobial components. Diethyl phthalate is therefore specifically excluded from the well characterized fragrance compositions of the invention.

Among the perfumery materials which although permitted as well characterized fragrance materials are known to have undesirable characteristics and are therefore preferably excluded from the invention perfume compositions are nitro musks as exemplified by musk ketone (CAS 81-14-1). Carbitol ethers defined as compounds of formula R-(OCH₂CH₂)ₙ -OR¹ where n= 1,2 or 3 R= (C₁ to C₇) alkyl or phenyl or alkyl substituted phenyl and R¹ is H or (C₁ to C₇)alkyl are preferably excluded.

It is also preferable if the levels of the following fragrance ingredients are limited to less than 0.1% by weight of the final product from all sources (including contributions from the optional natural extracts), preferably less than 0.01% by weight and more preferably less than 0.001% by weight of the final product: anisyl alcohol (CAS 105-13-5), benzyl benzoate (CAS 120-51-4), benzyl cinnamate (CAS 103-41-3), cinnamic aldehyde (CAS 104-55-2), cinnamyl alcohol (CAS 104-54-1), citronellal (CAS 106-22-9), coumarin (CAS 91-64-5), eugenol (CAS 97-53-0), geraniol (CAS 106-24-1), hydroxycitronellal (CAS 107-95-5), isoeugenol (CAS 97-54-1), lilial (CAS 80-54-6), limonene (CAS 5989-27-5), linalool (CAS 78-70-6).

### Fragrance level

Fragrance dosage depends on the type of product and some typical dosage levels are shown in table 5 below. However for preservative perfumes of the prior art, as demonstrated in example 1 of EP 0 570 794A2, high levels of fragrance may need to be dosed to preserve a product, because the antimicrobial effectiveness of the fragrance ingredients is generally much weaker than that of conventional preservatives. Well characterized fragrance ingredients of the current invention may be dosed at the same level as conventional fragrances. Indeed it may be preferred if the fragrance is dosed at lower levels than conventional fragrances typically below 1.0% by weight of the final product composition, preferably below 0.6% by weight of the final product composition, more preferably below 0.4% by weight of the final product composition and particularly preferably below 0.2% by weight of the final formulation.

### Preservatives

Compositions of the invention must also contain either one or more of the preservatives listed in table 3 or one or more of the chelants, (which may alternatively be termed sequestrants) listed. Depending on the particular product and relevant legislation, in force, the preservative or mixture of preservatives if used would normally be dosed at a level of about 0.005%-1.0% by weight of the preservative composition.

**Table 3: Preservatives**

| **COMPOUND NAME** | **CAS NO** |
|---|---|
| Formaldehyde and paraformaldehyde | 50-00-0, 30525-89-4 |
| Biphenyl-2-ol ( ortho phenylphenol) | 90-43-7 |
| 4,4-dimethyloxazolidine | 51200-87-4 |
| 5-bromo-5-nitro-1,3 dioxane (Bronidox^{®}) | 30007-47-7 |
| 2-bromo-2-nitropropane-1,3-diol (Bronopol^{®}) | 52-51-7 |
| 2,4 dichlorobenzyl alcohol | 1777-882-8 |
| 5-chloro-2-(2,4-dichlorophenoxy)-phenol (Triclosan^{®}) | 3380-34-5 |
| 4-Chloro-3,5-xylenol | 88-04-0 |
| 3,3'-bis (1-hydroxymethyl-2,5-dioxoimidazolidinyl-4-yl)-1,1'-methylenediurea (imidazolidinyl urea) | 39236-46-9 |
| Poly(1-hexamethylene biguanidine hydrochloride) | 32289-58-0 |
| 2-Phenoxyethanol | 122-99-6 |
| Hexamethylenetetramine | 100-97-0 |
| Benzyl alcohol | 100-51-6 |
| 1,3 Bis (hydroxymethyl)-5,5-dimethylimidazolidine -2,4-dione, DMDM hydantoin, Glydant ® | 6440-58-0 |
| Mixture of 5-chloro-2-methylisothiazol-3(2H)-one and 2-methyl-isothiazol-3(2H)-one (Kathon CG^{®}) | 26172-55-4/ 2682-20-4 |
| Benzisothiazolinone | 2634-33-5 |
| 2-benzyl-4 -chlorophenol | 120-32-1 |
| Chlorhexidine and its digluconate, diacetate, and dihydrochloride salts | 55-56-1 |
| 1-phenoxy-propan-2-ol | 770-35-4/ 4169-04-4 |
| Cetyl pyridinium bromide and chloride | 122-03-5 |
| N-(hydroxymethyl)-N-dihydroxymethyl-1,3-dioxo-2,5-imidazolinidyl-4)-N'-hydroxymethyl urea (Germall 115 ^{®}) | 39236-46-9 |
| Sodium hydroxymethylglycinate | 70161-44-3 |
| Benzethonium Chloride | 121-54-0 |
| Benzalkonium chloride, bromide and saccharinate | 8001-54-5 |
| 3-iodopropynylbutylcarbamate | 55406-53-6 |
| Benzisothiazolinone | 2634-33-5 |
| Triacetin | 102-76-1 |
| cis-1-(3-chloroallyl-3,5,7-triaza-1-azoniaadamantane chloride (Dowicil200^{®}) | 51229-78-8 |

More preferred as preservatives of the current invention are: benzyl alcohol, 2-phenoxyethanol, cis 1-(3-chloroallyl)-3,5,7-triaza-1-azoniaadamantane chloride, iodopropynyl butylcarbamate, benzalkonium chloride, 2 bromo-2-nitropropane-1,3-diol, 1,3-bis-(hydroxymethyl)-5,5-dimethylimidazolidine, benzisothiazolinone, 5-chloro-2-methylisothiazol-3(2H)-one, 2-methyl-isothiazol-3(2H)-one, cetyl pyridinium bromide and chloride, imidazolidinyl urea, diazolidinyl urea, poly(1-hexamethylene biguanidine hydrochloride).

Most preferred as preservatives of the current invention are: benzyl alcohol, 2-phenoxyethanol, cis 1-(3-chloroallyl)-3,5,7-triaza-1-azoniaadamantane chloride, iodopropynyl butylcarbamate, 2-bromo-2-nitropropane-1,3-diol, 1,3-bis-(hydroxymethyl)-5,5-dimethylimidazolidine, cetyl pyridinium bromide and chloride, imidazolidinyl urea, diazolidinyl urea, poly(1-hexamethylene biguanidine hydrochloride).

If utilised these materials would usually comprise 10-80% by weight of the preservative composition more preferably 20-80% by weight and even more preferably 30-80% by weight of the preservative composition and these preservatives would be dosed at 0.05% to 1.0% by weight of the final product composition.

The composition of the invention may optionally contain one or more of the preservatives listed in table 4 as optional preservatives. When used such optional preservatives would be dosed at a level of about 0.05% by weight to 1.0% by weight of the final product composition. The optional preservative may constitute an amount from 0-80% by weight of the preservative composition of the invention, preferably 0-60% by weight, more preferably 0-40% by weight and even more preferably 0-20% by weight of the preservative composition:

**Table 4: Optional Preservatives**

| **COMPOUND NAME** | **CAS NO** |
|---|---|
| Benzoic acid and its salts | 65-85-0 |
| Para hydroxybenzoic acid and its salts | 99-96-7 |
| Methyl parahydroxybenzoate* | 99-76-3 |
| Ethyl parahydroxybenzoate* | 120-47-8 |
| Propyl parahydroxybenzoate* | 94-13-3 |
| Butyl parahydroxybenzoate* | 94-26-8 |
| Isobutyl parahydroxybenzoate* | 4247-02-3 |
| Methyl benzoate | 92-58-4 |
| Ethyl benzoate | 93-89-0 |
| Iso butyl benzoate | 120-50-3 |
| Propionic acid and its salts | 79-09-4 |
| 3-acetyl-6-methylpyran-2,4-(3H)-dione (dehydroacetic acid) and its salts | 520-45-6 |
| Salicylic acid and its salts | 69-72-7 |
| Phenyl salicylate | 118-55-8 |
| Sorbic acid (hexa-2,4-dienoic acid) and its salts | 110-44-1 |
| Formic acid and its sodium salt | 64-18-6 |
| Undec-10-enoic acid and its salts | 112-38-9 |

| | |
|---|---|
| * parahydroxybenzoates are commonly named « parabens » | |

Salts of the organic acids include salts with metal cations, such as sodium, potassium, magnesium and calcium, and also with organic cations, such as ammonium salts and alkyl substituted ammonium salts, e.g. triethanolamine, monoethanolamine. The CAS numbers for organic acids given above refer to that of the parent acid; the salts have different CAS numbers. In the case of salicylic acid, salts have the following CAS numbers: 824-35-1, 18917-89-0, 59866-70-5, 578-36-9, 54-21-7, 2174-16-5.

Preferred optional preservatives parahydroxy benzoic acid and salts thereof, (C₁-C₄) alkyl parahydroxy benzoates, sorbic acid and salts thereof, ortho hydroxybenzoic acid and salts thereof, benzoic acid and salts thereof, (C₁-C₂) alkyl benzoates, and

Particularly preferred optional preservatives are (C₁-C₃) alkyl parahydroxy benzoates, sorbic acid and salts thereof, benzoic acid and salts thereof.

### Sequestrants

The compositions herein may optionally contain one or more transition metal chelating agents termed "chelators" or sequestrants. Such chelating agents can be selected from the group consisting of amino carboxylates, amino phosphonates, polyfunctionally-substituted aromatic chelating agents and mixtures thereof, all as hereinafter defined.

Amino carboxylates useful as chelating agents include ethylenediaminetetracetates, N-hydroxyethylethylenediaminetriacetates, nitrilotriacetates, ethylenediamine tetraproprionates, triethylenetetraaminehexacetates, diethylenetriaminepentaacetates, and ethanoldiglycines, alkali metal salts, ammonium, and substituted ammonium salts thereof and mixtures thereof.

Amino phosphonates are also suitable for use as chelating agents in the compositions of the invention when at least low levels of total phosphorus are permitted in the product compositions, and include ethylenediaminetetrakis (methylenephosphonates) (CAS 7651-99-2 for pentasodium salt), diethylene pentakis(methyl phosphonates) (CAS 68155-78-2 for the heptasodium salt) and hydroxyethylidenel,1-diphosphonates (CAS 29329-71-9 for the tetrasodium salt) all sold under the DEQUEST^{®} brand.

Polyfunctionally-substituted aromatic chelating agents are also useful in the invention compositions (See US Patent. 3,812,044). Preferred compounds of this type in acid form are dihydroxydisulfobenzenes such as 1,2-dihydroxy-3,5-disulfobenzene.

Preferred are the biodegradable chelators such as ethylenediamine disuccinate ("EDDS"), especially the [S, S] isomer as described in US Patent. 4,704,233, iminiodisuccinate (IDS)(CAS 144538-83-0 for the terasodium salt) known as Baypure CX100 (RTM), and 3-hydroxy-2,2'-iminodisuccinate (HIDS) (CAS 190195-65-4 for the tetrasodium salt) available from Nippon Shokubai, tetrasodium N,N-bis-(carboxylatomethyl)-L-glutamate (CAS 51981-21-6 for the tetra sodium salt) known under the trade name Dissolvine GL38® from (Akzo Nobel).

The compositions herein may also contain water-soluble methyl glycine diacetic acid (MGDA) salts (or acid form)

Polycarboxylic acids and their salts are a group of materials which function as sequestrants but also have other applications such as buffering or pH adjustment agents and as builders in laundry detergent products. Builders are calcium and magnesium sequestering agents such as polycarboxylates, for example citric acid and tartaric acid, polymers and copolymers containing carboxylic acid groups such as poly(maleic acid) and poly(acrylic acid) such as the Sokalan® range supplied by BASF, may be used at high levels in laundry liquids. Polyaspartic acid and salts therof is an example of a biodegradable polymer with multiple carboxylate groups.

Preferred chelating agents include polyacrylic acid and/or salts thereof, copolymers of acrylic and maleic acid and/or salts thereof, polyaspartic acid and/or salts thereof, ethylene diamine tetraacetic acid (EDTA) and/or salts thereof, citric acid and/or salts thereof, ethylenediaminedisuccinic acid and/or salts thereof, N,N-bis-(carboxylatomethyl)-L-glutamic acid and/or salts thereof, iminiodisuccinic acid and/or salts thereof and 3-hydroxy-2,2'-iminodisuccinic acid and/or salts thereof (HIDS). ethylenediaminetetrakis (methylene-phosphonic acid) acid and/or salts thereof, diethylene pentakis (methyl phosphonic acid) acid and/or salts thereof and hydroxyethylidene-1,1-diphosphonic acid and/or salts thereof and mixtures thereof.

Especially preferred chelating agents include ethylene diamine tetraacetic acid (EDTA) and/or salts thereof, citric acid and/or salts thereof, ethylenediaminedisuccinic acid and/or salts thereof, N,N-bis-(carboxylatomethyl)-L-glutamatic acid and/or salts thereof, polyacrylic acid and/or salts thereof, copolymers of acrylic and maleic acid and/or salts thereof and mixtures thereof.

If utilized, these chelating agents with the exception of the polycarboxylate sequestrants will generally comprise from about 0.05% to about 2.0% by weight of such final product compositions. Polycarboxylate sequestrants may comprise from 0.05% to 6.0% by weight of a final product composition.

### Optional Natural Extracts

Compositions of the present invention may optionally contain natural extracts, such as essential oils. Natural extracts are produced by subjecting suitable natural materials such as plant components: leaves, flowers, seeds, roots or stems to an extraction process. The extraction processes are well known to those skilled in the art and are described in The Essential Oils by E Guenther published in 1949 by D van Nostrand. Essential oils can undergo additional processes to rectify and purify the oils for example by removing the terpene components via a "head cut" and/or removing the wax components via a "tail cut". Such natural extracts include but are not limited to those obtained from citrus species such as: lemons, oranges, mandarin, grapefruit, ugli fruit, anise, clove, basil, aniseed, cinnamon, geranium, roses, mint, lavender, lavandin, thyme, rosemary, citronella, cypress, eucalyptus, peppermint, Peru balsam, camphor, sandalwood, ylang and cedarwood and mixtures thereof. A preferred group of natural extracts for the present invention are Amyris oil, cedarwood oil, cocoa absolute, copaiba balsam, menthe oil pays, myrrh resin, patchouli oil, vanillin (absolute) and vetiver oil.

Natural extracts are frequently used in cosmetic and personal care products for their many benefits including their fragrance. Also many natural extracts are reported as having antimicrobial properties as mentioned earlier in the present invention. While the antimicrobial properties of natural extracts may assist product preservation they are primarily be added to the composition for their contribution to fragrance or other cosmetic benefit.

### Products to be preserved

Products to be preserved according to the invention, also named final products in the present description, are personal care, household and laundry compositions which are not intended for human or animal ingestion. Included within the definition of products to be ingested for purposes of the present invention are products for dental and oral care, such as toothpastes and mouth washes which although not intended for ingestion may nevertheless accidentally enter the gastro-intestinal tract.

Personal care compositions include creams, emulsions, lotions gels and oils for the skin (face, hands, feet etc) tinted bases (liquids and pastes) deodorant and antiperspirant products, products for removing make-up from the face and eyes. Hair care products include: hair tints and bleaches, products for waving, straightening, setting and fixing hair. Shaving products, including creams, foams and depilatory products, sun bathing products and products for tanning without the sun. Personal cleansing products for the skin include toilet soaps, deodorant soaps, bath and shower preparations (salts, foams, lotions, liquids oils, gels etc.). Hair cleansing products include shampoos and conditioners.

Household cleansing preparations include hand dish wash liquids, general purpose cleansers, liquids, mousses, creams and abrasive liquids and freshener sprays. Laundry liquid detergents, fine wash products, fabric softeners and fabric conditioners and moist tumble drier sheets.

Products to be preserved will contain a certain proportion of water, generally at least 15% by weight, in many cases at least 30% by weight and in most products more than 60% by weight or even higher proportions. Furthermore, such products will usually contain some surface active materials, either as an emulsifier, if the product is an emulsion, or as a detergent active material if the product has some kind of cleaning activity.

Generally, the concentration of surface active material in the product will be 0.1-60% by weight; usually the level of surface active material will be 50% by weight or lower; for most products the level of surface active material will be 30% by weight or lower. On the other hand, the level of surface active material will usually be at least 0.1% by weight preferably greater than 1.0% and more preferably greater than 3.0% by weight. For products which have a cleaning function it is likely the level of surface active material will be higher typically greater than 10% by weight and preferably greater than 15% by weight. Examples of leave-on products containing emulsifiers are: hand and body lotions, make up removing lotions, skin creams, sunscreen products and sunless tanning products and domestic freshener sprays. Also included are articles of manufacture impregnated with liquids, for example pads or wipes impregnated with lotions for make up application or removal, or to apply sunscreen compounds or sunless tanning agents, for personal cleansing e.g. as moist toilet tissue or baby wipes which are considered leave-on products. Examples of cleansing products containing detergents are: shampoos, body washes, dishwashing liquids, laundry liquids, general purpose cleaners, liquid abrasive cleaners, liquid soaps, fabric softeners. Again articles or substrates such as pads, sponges or wipes made from non woven textiles, may be impregnated with liquids for cleaning inanimate surfaces such as kitchen worktops, tiled surfaces, bathroom sanitary ware, windows, leather goods and soft furnishings. Some products may fall into more than one category.

Final products containing preservative compositions of the present invention typically comprise from about 15% by weight to about 95% by weight, preferably from about 25% by weight to about 90% by weight, more preferably from about 30% by weight to about 80% by weight of water.

The pH of the products containing preservative compositions of the invention is important to product preservation but it is also important to the physical stability and function of the product. For Household and laundry cleaning products the pH can be in the range of from 2 to about 10, preferably from about 3 to about 9. For cosmetic toiletry and personal care products the pH is preferably from about 4 to about 8, and more preferably from about 5.5 to about 7.5.

Typical quantities of water, surface active material and perfume, listed as weight percentage, in different kinds of products are set out in table 5 below.

**Table 5**

| Product | Surface active material (%) | Water (%) | Perfume (%) |
|---|---|---|---|
| Oil-in-water skin cream | 10 | 60 | 0.2 |
| Water-in-oil skin cream | 2 | 60 | 0.4 |
| Eye make-up remover | 5 | 60 | 0.2 |
| Liquid abrasive cleaner | 12 | 32 | 0.3 |
| General purpose cleaner | 8 | 90 | 0.3 |
| Shampoo | 20 | 75 | 0.5 |
| Body Wash | 15 | 80 | 1.0 |
| Cleansing Wipes* | 5 | 90 | 0.2 |
| Lotion skin Wipes | 5 | 80 | 0.1 |
| Window cleaner | 0.2 | 90 | 0.1 |
| Fabric softener | 5 | 94 | 0.4 |
| Hair conditioner | 5 | 90 | 0.5 |
| Dishwashing liquid | 40 | 55 | 0.2 |
| Heavy laundry liquid | 15 | 55 | 0.4 |

| | | | |
|---|---|---|---|
| * figures based on composition of liquid used to impregnate the wipe. | | | |

The formulations and ingredients of household, laundry and personal care products and cosmetics in which preservative compositions of the invention may be used are well known to those skilled in the art, reference may be made to the following works which are incorporated herein by reference:
"Formulating Detergents and Personal Care Products A guide to Product Development" by L Ho Tan Tai, ISBN 1-893997-10-3 published by the AOCS Press, and
also to Surfactant Science Series Volume 67 "Liquid Detergents" ISBN 0-8247-9391-9 (Marcel Dekker Inc) and
"Surface Active Agents and Detergents" Volumes 1 and 2 by Schwartz, Perry and Birch, and
"Harry's Cosmeticology" published by CHS Press 8th Edn 2000 ISBN 0-8206-0372-4, and
Mc Cutcheon's Detergents and Emulsifiers Published by Allured.
Cosmetic Science and Technology Series Volume 20, Edited by K.Laden, (Marcel Dekker) ISBN 0-8247-1746-5 "Antipersperants and Deodorants".
Surfactant Science Series Volume 71,"Powdered Detergents" ed M Showell ISBN 0-8247-9988-7
Cosmetic Science and Technology Series Volume 17, Ed by Dale H Johnson, ISBN 0-8247-9365-X "Hair and Hair Care" published by Marcel Dekker, as well as to the following patents or patent applications; all of these patents and the references therein are incorporated herein by reference.
Fabric softeners, conditioners and post wash treatments:
US6,335,315; US5,674,832; US5,759,990; US5,877,145; US5,574,179; US4767547; US6806248; EP1,141,188; EP1,287,198; EP0,459,822; EP0,392,606.
Liquid laundry detergents:
US5,929,022; US5,916,862; US5,731,278; US5,470,507; US5,466,802; US5,460,752 and US5,458,810 and WO20010,053,754; WO2006,053,615.
Shampoos and Hair Conditioners:
US6,162,423; US5,968,286; US5,935,561; US5,932,203; US5,837,661; US5,776,443; US5,756,436; US5,661,118; US5,618,523; US2004021912; EP0018717; EP1009365; EP0200305.
Wipes
WO2003051327; WO0004230, EP1361855; US2005/0008680;
Skin cleansing and care liquids
US5,833,999; EP1066827; EP1510201; EP573229; US2005/0085405;

The present compositions are used in a conventional manner for cleaning the skin and/or hair and to provide olfactory aesthetic benefit. An effective amount of the product, typically from about 1 g to about 15 g of the composition, is applied to the body or hair that has preferably been wetted, generally with water. Applications includes dispensing of the composition onto the hand, onto the body or hair, or onto a washing implement, e.g., wash cloth, sponge, etc., and typically includes working the composition with the hands to develop lather. The lather can stand on the body for a length of time or can be rinsed immediately with water. Once the product is rinsed from the body the washing procedure can be repeated.

### Examples: Fragrance compositions suitable for the purposes of the invention

### Example 1: Composition of Fragrance A

| **Ingredient** | **% (by weight)** |
|---|---|
| Allyl caproate | 1.19 |
| Dihydrojasmonate | 21.43 |
| Dipropylene Glycol | 29.76 |
| Geranyl acetate | 7.14 |
| Heliotropine | 16.66 |
| Ethylene brassylate | 23.81 |

### Example 2: Composition of Fragrance B

| **Ingredient** | **% (by weight)** |
|---|---|
| Benzaldehyde | 1.0 |
| Dihydrojasmonate | 20 |
| Dipropylene Glycol | 33 |
| Cis hex-3 en-1-ol | 1 |
| Santalex T | 13.2 |
| Ethylene brassylate | 30 |
| Iso propyl myristate | 1.8 |

### Example 3: Composition of fragrance C

| **Ingredient** | **% (by weight)** |
|---|---|
| Dihydrojasmonate | 18.50 |
| Ethyl vanillin | 3.70 |
| Santalex T | 5.43 |
| Isopropyl myristate | 0.76 |
| Heliotropine | 22.22 |
| Ethylene brassylate | 49.38 |

### Examples 4 to 9: Composition of fragrance E to J

| Ingredients | Fragrance | | | | | |
|---|---|---|---|---|---|---|
| | E | F | G | H | I | J |
| | Ex. 4 | Ex.5 | Ex.6 | Ex.7 | Ex.8 | Ex.9 |
| Undecalactone gamma | | 20 | | 10 | | |
| Nonalactone gamma | | | | | 5 | |
| Benzyl acetate | 10 | | | | 5 | |
| Benzaldehyde | | | 5 | | | 2 |
| Ethyl vanillin | 10 | 10 | 22 | 10 | | |
| Geranyl acetate | | 30 | | | | |
| Heliotropine | 50 | | | | 30 | |
| Ethylene brassylate | 30 | 40 | | 60 | 60 | 53 |
| Santalex T | | | 64 | | | 40 |
| Verdox | | | | 20 | | |
| Isopropyl myristate | | | 9 | | | 5 |

### Test examples

### Anti-Bacterial Test Method

Materials were dissolved in ethanol (or deionized water in the case of water soluble preservatives) to a concentration of 100 mg/ml. 30 µl of this solution added to 3 ml deionized water gave a final concentration of 0.1%. (If necessary, solutions are sonicated for 15 minutes and allowed to equilibrate back to ambient temperature.) Bacterial cultures were added to this solution, mixed on a vortex mixer and a clock started. After 3 days, an aliquot is sampled and added to a D/E (Dey/Engley) neutralization broth (marketed by Becton and Dickinson Cy) and plated onto solid media. Bacterial colonies are counted after overnight incubation. The selected indicator microorganism was *Pseudomonas aeruginosa* ATCC 15442.

All results are quoted as the reduction in activity expressed as a logarithm to the base 10 measured after 3 days incubation and compared with the inocculum unless otherwise noted.

All percentages are quoted as weight per unit volume (e.g. g/ml) unless indicated otherwise.

### Test example 1

In this example, a preservative plus an individual fragrance is compared with a mixture containing the same preservative plus half the dosage of each of the two fragrance ingredients together. The combination of 2 fragrance materials is better than either one.

| **Test material** | **log reduction after 3 days vs Inocculum** |
|---|---|
| 0.1%*Propyl paraben +0.1%Ethylene brassylate | 0 |
| 0.1%Propyl paraben +0.1%Ailyl caproate | 0 |
| 0.1%Propyl paraben +0.05%Ethytlene brassylate +0.05%Allyl caproate | 1.7 |

| | |
|---|---|
| *0.1% = 0.1g/100ml | |

### Test example 2

In this example, a mixture of preservative with one fragrance ingredient and an optional natural extract is compared with a mixture of preservative with two fragrance ingredients and the same natural extract. Addition of the second material increases the antibacterial effect.

| **Test material** | **log reduction after 3 days vs Inocculum** |
|---|---|
| 0.1%Propyl paraben +0.05%Ethyl acetate +0.05% Cedarwood oil | 0 |
| 0.1%Propyl paraben +0.025%Ethyl acetate +0.025% Cedarwood oil +0.05% Allyl caproate | 1.6 |

### Test example 3

A mixture of a sequestrant and two fragrance ingredients is compared with the same mixture containing a third fragrance ingredient. Again there is a substantial improvement in antibacterial efficacy from addition of a third fragrance ingredient.

| **Test material** | **log reduction after 3 days vs Inocculum** |
|---|---|
| 0.1% EDTA +0.05% Ethylene brassylate +0.05% Santalex T | 0 |
| 0.1% EDTA +0.025% Ethylene brassylate +0.025% Santalex T +0.05%Benzaldehyde | 3.6 |

### Test example 4

This example shows the improved antibacterial effect of a combination of Dowicil 200 and fragrance A, a fragrance containing 6 ingredients selected according to the invention compared with Dowicil 200 at the same dosage.

| **Test material** | **Log reduction after 3 days vs 0.05% Dowicil 200 alone** |
|---|---|
| 0.2% **Fragrance A** + 0.05% Dowicil 200 | >4 |

### Test example 5

This test example repeats test example 4 but using phenoxyethanol as the preservative instead of Dowicil 200 and showing improved antibacterial efficacy for the combination.

| **Test material** | **Log reduction after 3 days vs 0.1% phenoxyethanol alone** |
|---|---|
| 0.1% Fragrance A + 0.1% Phenoxyethanol | 1.9 |
| 0.2% Fragrance A + 0.1% Phenoxyethanol | 2.3 |

### Test example 6

This test example repeats example 5 but with fragrance B instead of Fragrance A and confirming the antibacterial efficacy of the combination.

| **Test material** | **Log Reduction after 3 days vs 0.1% Phenoxyethanol** |
|---|---|
| 0.2%Fragrance B + 0.1% Phenoxyethanol | >4 |

### Test example 7

This example shows the improved antibacterial effect of combining fragrance A with a preservative and citric acid as sequestrant compared with citric acid alone.

| **Test material** | **Log Reduction after 3 days vs 0.025% Citric Acid** |
|---|---|
| 0.2% Fragrance A + 0.06% Phenoxyethanol + 0.025% Citric acid | 2.4 |

### Test example 8

This test example compares the antibacterial efficacy of 3 different fragrances, formulated according to the invention and in admixture with 0.1% phenoxyethanol. A dose of 0.1% phenoxyethanol in the same test gave 0.4 log reduction, compared with the ethanol solvent control, after 3 days the combination of fragrance and preservative is always superior.

| **Perfume** | **0.2% perfume** | **0.1% perfume + 0.1% Phenoxyethanol** |
|---|---|---|
| | log reduction after 3 days | log reduction after 3 days |
| Fragrance F | 0.8 | 2.4 |
| Fragrance H | 0.7 | 2.4 |
| Fragrance J | 0.4 | 2.3 |

### Test example 9

This test example 9 repeats test example 8, but replacing the 0.1% phenoxyethanol with 0.05% Dowicil 200. Thus in every case the perfume and Dowicil combination is more effective than either perfume or Dowicil alone. A dose of 0.05% Dowicil 200 in the same test gave 1.3 log reduction, compared with the ethanol solvent control.

| **Perfume** | **0.2% perfume** | **0.1% perfume + 0.05% Dowicil 200** |
|---|---|---|
| | log reduction after 3 days | log reduction after 3 days |
| Fragrance E | 0 | >4 |
| Fragrance G | 0 | >4 |
| Fragrance I | 0 | >4 |

### Test example 10

In test example 10, two preservative compositions of the invention containing three components selected from a), b), c) and d) were tested. The two combinations show increased reduction in antibacterial efficacy especially for the replacement of a preservative by a sequestrant.

| a) | b) | c) | d) | log reduction after 3 days vs Inocculum |
|---|---|---|---|---|
| 0.025% DHiJ* 0.05% Allyl Caproate | 0.1% propyl paraben | 0.025% Phenoxyethanol | | 2.5 |
| 0.025% DHiJ* 0.05% Allyl Caproate | | 0.025% Phenoxyethanol | 0.025% citric acid | >4 |

| | | | | |
|---|---|---|---|---|
| * DHiJ = Dihydrojasmonate | | | | |

### Test example 11

The antibacterial effects of a preservative composition comprising a), b), c), or a), and c) of the invention are shown to be effective.

| a) | b) | c) | d) | log reduction after 3 days vs Inocculum |
|---|---|---|---|---|
| 0.1% fragrance A | 0.1% propyl paraben | 0.13% Phenoxyethanol | 0.025% Citric acid | >4 |
| | | 0.05% Dowicil | | 0.7 |
| 0.1% fragrance A | | 0.05% Dowicil | | >4 |

### Test example 12

This test example shows that addition of diethyl phthalate is detrimental to antibacterial efficacy.

| **Test material** | **log reduction after 3 days vs Inocculum** |
|---|---|
| 0.05% allyl Caproate +0.1% Propyl Paraben +0.05% Phenoxyethanol | 2.4 |
| 0.05% allyl Caproate +0.1% Propyl Paraben +0.05% Phenoxyethanol +0.1% Diethyl phthalate | 1.9 |

### Test example 13

This test example again proves that addition of diethyl phthalate is detrimental to preservative efficacy.

| **Test material** | **log reduction after 3 days vs Inocculum** |
|---|---|
| 0.05% heliotropine +0.05% Propyl Paraben | 0.9 |
| 0.05% heliotropine +0.05% Propyl Paraben +0.2% Diethyl phthalate | 0.4 |

The following examples 10 to 17 are examples of final products containing a preservative composition according to the invention

### Example 10

This example is a shampoo final product incorporating a preservative composition of the invention containing fragrance A of example 1 along with EDTA sequestrant and salicylic acid and sodium benzoate preservatives fulfilling the requirements of the invention.

| **Ingredient** | **Wt %** |
|---|---|
| Lauryl ether sulphate | 14.0 |
| Cocoamidopropyl betaine | 6.5 |
| Glycerol | 2.0 |
| Sodium N-cocoylamidoethyl N-ethoxycarboxymethylglycinate | 2.0 |
| Coconut Monoethanolamide | 0.8 |
| Copolymer of dimethyldiallyl ammonium chloride and acrylamide | 1.5 |
| Salicylic Acid | 0.2 |
| Sodium Benzoate | 0.5 |
| Disocdium Ethylene diamine tetra-acetate | 0.25 |
| Perfume A of example 1 | 0.2 |
| Ethylene glycol distearate | 0.2 |
| EDTA | 0.25% |
| Ph adjust with lactic acid solution or Sodium hydroxide solution | To pH 5.2 |
| Water | To 100 |

### Example 11

Example 11 is a leave-on facial emulsion composition containing a cationic hydrophobic surfactant which is prepared by combining the following components utilizing conventional mixing techniques.
The composition contains 0.06% fragrance B, plus 0.1% of tetrasodium EDTA and 0.1% of DMDM hydantoin thus complying with the requirements of the invention.

| **Ingredient** | **Wt(%)** |
|---|---|
| Water to | 100 |
| Glycerin | 3.00 |
| Cetyl Palmitate | 3.00 |
| Cetyl Alcohol | 1.26 |
| Quaternium-22*** | 1.00 |
| Glyceryl Monohydroxy Stearate | 0.74 |
| Dimethicone | 0.60 |
| Stearic Acid | 0.55 |
| Octyldodecyl Myristate | 0.20 |
| Fragrance B of example 2 | 0.06 |
| Carbomer 1342* | 0.125 |
| Tetrasodium EDTA | 0.10 |
| DMDM Hydantoin** | 0.10 |
| Carbomer 951* | 0.075 |

| | |
|---|---|
| *supplied by Noveon Inc Ohio USA ** supplied by Brentag, Sartrouville, France *** supplied by Seppic, Paris, France | |

### Example 12

This example is an aqueous skin cleansing liquid for impregnating a non woven textile to form a moist wipe which contains a fragrance according to the invention.

The composition contains 0.2% fragrance A, 0.1% Dissolvine GL38 and 1.0% Germaben II hence complying with the requirements of the invention.

| **Ingredient dosage** | **Wt(%)** |
|---|---|
| Propylene Glycol | 0.8 |
| Polysorbate 20 | 1.5 |
| Germaben II (RTM) | 1.0 |
| Fragrance A of example 1 | 0.2 |
| Dissolvine GL38 | 0.1 |
| Silicone antifoam 1510* | 0.015 |
| Lactic acid | To pH 5.4 |
| Water | To 100% |

| | |
|---|---|
| * supplied by Univar, Fontenay-sous-Bois, France. | |

Germaben II is a commercial preservative from ISP containing diazolidinyl urea methyl paraben and propyl paraben. Various additives could be added to such a formulation for skin benefit such as Aloe vera, D,L-panthenol, chamomile extracts in which case the water content would be adjusted to accommodate the additives. Such a liquid would be dosed at around 125gm⁻² on 50gm⁻² spun lace non woven substrate.

### Example 13

This example is a phase inversion emulsion composition containing a fragrance of the invention for impregnating onto a non woven fabric as a moist wipe and incorporating a fragrance according to the invention.

| **Ingredient** | **Wt (%)** |
|---|---|
| Emulgade CM(RTM) | 15 |
| Ceteareth 20 | 4.7 |
| Dicapryl ether | 4.0 |
| Cetearyl isononanoate | 5.0 |
| Cocoglycerides | 2.0 |
| Fragrance A of example 1 | 0.15 |
| Euxyl K702 | 1.0 |
| Tetrasodium EDTA | 0.1 |
| Citric acid | 0.04 |
| Water | To 100 |

Emulgade CM is a concentrated emulsion of cosmetic oils and non-ionic emulsifiers which dilutes into a phase inverted emulsion mixture supplied by Cognis.
Ceterareth 20 is a non-ionic emulsifier.
Euxyl K702 is a commercial mixture of preservatives from Schulke & Mayr containing Benzoic acid, dehydroacetic acid, phenoxyethanol, ethylhexylglycerin and polyaminopropyl biguanidine.
As in example 13 various beneficial additives can be incorporated into the formulation which would typically be dosed at around 125gm⁻² on 50gm⁻² spun lace non woven substrate. The composition contains 0.15% fragrance, 1.0%, preservative Euxyl K702 and 0.14% sequestrant, comprising 0.1% tetrasodium EDTA and 0.04% citric acid again fulfilling the requirements of the invention.

### Example 14

This example describes a household cleaner composition containing a fragrance of the invention.

| **Ingredient** | **Wt %** |
|---|---|
| Emulgin HF70 | 16.7 |
| 1,2 Propylene glycol | 4.0 |
| Perfume B of example 2 | 0.2 |
| Kathon CG | 0.005 |
| Natrosol 250MR | 11.7 |
| Dyes and other minors | q.s. |
| Water | To 100 |

Emulgin HF70 is supplied by Cognis
Kathon CG is a preservative mixture supplied by Seppic comprising methylchloroisothiazolinoneand methyl isothiazolinone.
Natrosol 250MR is supplied by Hercules.

### Examples 15-19: liquid Detergent Compositions

Examples 15 and 16 are standard liquid detergents, while examples 17 to 19 are concentrated liquid detergents, all contain fragrances of the invention.

| **Ingredient** | **Standard Liquid** | | **Concentrate Liquid** | | |
|---|---|---|---|---|---|
| | Ex. 15 Wt % | Ex. 16 Wt % | Ex.17 Wt % | Ex. 18 Wt % | Ex. 19 Wt % |
| Na-LAS | | | 14 | | |
| Na-PAS | 4 | 10 | 4 | 5 | 15 |
| Na-AES | | 2 | | | 2.6 |
| Nonionic 7EO | 9 | 3.5 | 15 | 24 | 5 |
| Cationic | | 1 | | | 2 |
| Soap | 15 | 7 | 12 | 17 | 11 |
| APG | 4 | | | 4 | |
| Glucosamide | | 4.5 | | | 6 |
| Mono ethanol amine | | 5 | | | 6.5 |
| Propylene glycol | 3 | 6 | 5 | | 8 |
| Glycerol | | | | 4 | |
| Ethanol | 2 | 1 | | 7 | 2.2 |
| Citrate | 1 | 2 | 6 | 1 | 2 |
| Perfume C of example 3 | 0.2 | 0.2 | 0.5 | 0.5 | 0.6 |
| Benzisothiazolinone | 0.05 | 0.05 | 0.05 | | |
| Kathon CG^{®} | | | | 0.05 | 0.05 |
| Viscosity modifier | | 0.25 | 0.18 | | 0.18 |
| Anti-foaming agent (15% active material) | 0.7 | 0.7 | 1 | 1 | 1 |
| Fluorescence agent (15% active material) | 0.7 | 0.7 | 1 | 1 | 1 |
| Sequestrating agent (Dequest® 2040 and 2010) | 1 | 1.4 | 1.5 | 1.5 | 1.5 |
| Enzymes (protease, lipase, cellulase, amylase) | 1.05 | 1.05 | 1.2 | 1.2 | 1.2 |
| Polymers for fabric maintenance ( elimination of stains ) | 1 | 1 | 1.5 | 1.5 | 1.5 |
| Water | to 100 | to 100 | to 100 | to 100 | to 100 |

### Examples 20 to 23

Examples 20-23 are dilute and concentrated liquid fabric conditioner compositions containing a fragrance of the invention, part of which may be encapsulated and which may, but need not, have the same composition as the free fragrance.

| Ingredient | Example 20 Dilute Fabric Conditioner | Example 21 Dilute Fabric Conditioner | Example 22 Concentrated Fabric Conditioner | Example 23 Concentrated Fabric Conditioner |
|---|---|---|---|---|
| | Wt % | Wt % | Wt % | Wt % |
| Tetranyl AHT-1 | 5.0 | | 12.0 | |
| Deqa | | 7.0 | | 18 |
| Genapol C200 | 0.1 | | 0.75 | 3 |
| Isopropyl alcohol | | 2.0 | | 3 |
| Polyethylene glycol 4000 | | | | 0.6 |
| Laurex CS | 0.4 | | 1.8 | |
| Fragrance of example a | 0.32 | 0.3 | 0.45 | 0.6 |
| Ca or Mg chloride | qs | qs | qs | qs |
| Dye, antifoaming agent, | qs | qs | qs | qs |
| Kathon CG^{®} | 0.10 | | 0.20 | |
| Benzisothiazolinone | | 0.10 | | 0.20 |
| Water to | 100 | 100 | 100 | 100 |

Tetranyl AHT-1 ; semi-hard tallow ester of triethanolammonium methosulphate, marketed by Kao Corp
Genapol C200 ; copra ethoxylate, marketed by Clariant
Laurex CS ; Long chain alcohol, marketed by Albright & Wilson
DEQA ; soft di(tallow oxyethyl) dimethylammonium chloride

## Claims

1. A preservative composition comprising:
a) 5-80% by weight of a mixture of 2-10 well **characterized** fragrance raw materials with a cosmetic function, of which at least 2 are selected from:
allyl caproate, benzyl acetate, benzaldehyde, dihydroisojasmonate, ethyl phenethylacetal, ethyl cinnamate, ethyl methyl phenyl glycidate, ethyl vanillin, 2-heptylcyclopentanone, geranyl acetate, heliotropine, cis hex-3-en-1-ol, ethylene brassylate, nonalactone gamma, camphylcyclohexanol, undecalactone gamma, 2-t-butylcyclohexylacetate, pentyl salicylate, 2-phenylethanol and 2-phenylethyl acetate
and represent at least 20% by weight of the said mixture, and
b) 0-80% by weight of one or more of the following preservatives:
parahydroxy benzoic acid and salts thereof, (C₁-C₄) alkyl parahydroxy benzoates, sorbic acid and salts thereof, ortho hydroxybenzoic acid and salts thereof, benzoic acid and salts thereof, (C₁-C₄) alkyl benzoates, propionic acid and salts thereof, dehydroacetic acid and salts thereof, formic acid and salts thereof, undec-10-enoic acid and salts thereof, and
either c) 10-80% by weight of one or more of the following preservatives:
formaldehyde, paraformaldehyde, biphenyl-2-ol (ortho phenylphenol), 4,4-dimethyloxazolidine, 5-bromo-5-nitro-1,3 dioxane, 2-bromo-2-nitropropane-1,3-diol, 2,4 dichlorobenzyl alcohol, 5-chloro-2-(2,4-dichlorophenoxy)-phenol, 4-chloro-3,5-xylenol, 3,3'-bis (1-hydroxymethyl-2,5-dioxoimidazolidinyl-4-yl)-1,1'-methylenediurea (imidazolidinyl urea), poly(l-hexamethylene biguanidine hydrochloride), 2-phenoxyethanol, hexamethylenetetramine, benzyl alcohol, 1,3 Bis (hydroxymethyl)-5,5-dimethylimidazolidine -2,4-dione, 5-chloro-2-methylisothiazol-3(2H)-one, 2-methyl-isothiazol-3(2H)-one, benzisothiazolinone, 2-benzyl-4 -chlorophenol, Chlorhexidine and its digluconate, diacetate, and
dihydrochloride salts, 1-phenoxy-propan-2-ol, cetyl pyridinium bromide and chloride, N-(hydroxymethyl)-N-dihydroxymethyl-1,3-dioxo-2,5-imidazolinidyl-4)-N'-hydroxymethyl urea, sodium hydroxymethylglycinate, benzethonium chloride, Benzalkonium chloride, bromide and saccharinate, 3-iodopropynylbutylcarbamate, benzisothiazolinone, triacetin, cis-1-(3-chloroallyl-3,5,7-triaza-1-azoniaadamantane chloride,
and/or d) 1-90% by weight of one or more of sequestrants selected among the following:
amino carboxylates, amino phosphonates, polyfunctionally-substituted aromatic chelating agents and polycarboxylic acids and salts thereof;
wherein the preservative composition optionally contains e) 0.1-50% by weight of one or more natural extracts, and the sum of a)-e) must equal 100% by weight.

2. A preservative composition according to claim 1, comprising:
a) 5-80% by weight of a mixture of 2-10 well **characterized** fragrance raw materials with a cosmetic function, of which at least 2 are selected from:
allyl caproate, benzyl acetate, benzaldehyde, dihydroisojasmonate, ethyl phenethylacetal, ethyl cinnamate, ethyl methyl phenyl glycidate, ethyl vanillin, 2-heptylcyclopentanone, geranyl acetate, heliotropine, cis hex-3-en-1-ol, ethylene brassylate, nonalactone gamma, camphylcyclohexanol, undecalactone gamma, 2-t-butylcyclohexylacetate, pentyl salicylate, 2-phenylethanol and 2-phenylethyl acetate
and represent at least 20% by weight of the said mixture, and
b) 0-80% by weight of one or more of the following preservatives:
parahydroxy benzoic acid and salts thereof, (C₁-C₄) alkyl parahydroxy benzoates, sorbic acid and salts thereof, ortho hydroxybenzoic acid and salts thereof, benzoic acid and salts thereof, (C₁-C₄) alkyl benzoates, propionic acid and salts thereof, dehydroacetic acid and salts thereof, formic acid and salts thereof, undec-10-enoic acid and salts thereof, and
either c) 10-80% by weight of one or more of the following preservatives:
benzyl alcohol, 2-phenoxyethanol, cis 1-(3-chloroallyl)-3,5,7-triaza-1-azoniaadamantane chloride, iodopropynyl butylcarbamate, benzalkonium chloride, 2 bromo-2-nitropropane-1,3-diol, 1,3-bis-(hydroxymethyl)-5,5-dimethylimidazolidine, benzisothiazolinone, 5-chloro-2-methylisothiazol-3(2H)-one, 2-methyl-isothiazol-3(2H)-one, cetyl pyridinium bromide and chloride, imidazolidinyl urea, diazolidinyl urea, poly(1-hexamethylene biguanidine hydrochloride);
and/or d) 1-90% by weight of one or more of sequestrants selected among the following:
amino carboxylates, amino phosphonates, polyfunctionally-substituted aromatic chelating agents and polycarboxylic acids and salts thereof;
wherein the preservative composition optionally contains e) 0.1-50% by weight of one or more natural extracts, and the sum of a)-e) must equal 100% by weight.

3. A preservative composition according to claim 1 or 2, comprising:
a) 5-80% by weight of a mixture of 2-10 well **characterised** fragrance raw materials with a cosmetic function, of which at least 2 are selected from:
allyl caproate, benzyl acetate, benzaldehyde, dihydroisojasmonate, ethyl phenethylacetal, ethyl cinnamate, ethyl methyl phenyl glycidate, ethyl vanillin, 2-heptylcyclopentanone, geranyl acetate, heliotropine, cis hex-3-en-1-ol, ethylene brassylate, nonalactone gamma, camphylcyclohexanol, undecalactone gamma, 2-t-butylcyclohexylacetate, pentyl salicylate, 2-phenylethanol and 2-phenylethyl acetate;
and represent at least 20% by weight of the said mixture, and
b) 0-80% by weight of one or more of the following preservatives:
parahydroxy benzoic acid and salts thereof, (C₁-C₄) alkyl parahydroxy benzoates, sorbic acid and salts thereof, ortho hydroxybenzoic acid and salts thereof, benzoic acid and salts thereof, (C₁-C₄) alkyl benzoates, propionic acid and salts thereof, dehydroacetic acid and salts thereof, formic acid and salts thereof, undec-10-enoic acid and salts thereof, and
either c) 10-80% by weight of one or more of the following preservatives:
benzyl alcohol, 2-phenoxyethanol, cis 1-(3-chloroallyl)-3,5,7-triaza-1-azoniaadamantane chloride, iodopropynyl butylcarbamate, benzalkonium chloride, 2 bromo-2-nitropropane-1,3-diol, 1,3-bis-(hydroxymethyl)-5,5-dimethylimidazolidine, benzisothiazolinone, 5-chloro-2-methylisothiazol-3(2H)-one, 2-methyl-isothiazol-3(2H)-one, cetyl pyridinium bromide and chloride, imidazolidinyl urea, diazolidinyl urea, poly(l-hexamethylene biguanidine hydrochloride)
and/or d) 1-90% by weight of one or more of the following sequestrants:
ethylenediamine tetraacetic acid and salts thereof, citric acid and salts thereof, ethylenediamine disuccinic acid and salts thereof, tetrasodium glutamate diacetate,
wherein the preservative composition optionally contains e) 0.1-50% by weight of one or more natural extracts, and the sum of a)-e) must equal 100% by weight.

4. A preservative composition according any one of claims 1 to 3, which comprises:
a) 5-80% by weight of a mixture of 4-10 well **characterised** fragrance raw materials with a cosmetic function, of which at least 4 materials are selected from:
allyl caproate, benzyl acetate, benzaldehyde, dihydroisojasmonate, ethyl phenethylacetal, ethyl cinnamate, ethyl methyl phenyl glycidate, ethyl vanillin, 2-heptylcyclopentanone, geranyl acetate, heliotropine, cis hex-3-en-1-ol, ethylene brassylate, nonalactone gamma, camphylcyclohexanol, undecalactone gamma, 2-t-butylcyclohexylacetate, pentyl salicylate, 2-phenylethanol and 2-phenylethyl acetate
and represent at least 20% by weight of the said mixture, and
b) 0-80% by weight of one or more of the following preservatives:
parahydroxy benzoic acid and salts thereof, (C₁-C₄) alkyl parahydroxy benzoates, sorbic acid and salts thereof, ortho hydroxybenzoic acid and salts thereof, benzoic acid and salts thereof, (C₁-C₄) alkyl benzoates, propionic acid and salts thereof, dehydroacetic acid and salts thereof, formic acid and salts thereof, undec-10-enoic acid and salts thereof, and
either c) 10-80% by weight of one or more of the following preservatives
benzyl alcohol, 2-phenoxyethanol, cis 1-(3-chloroallyl)-3,5,7-triaza-1-azoniaadamantane chloride, iodopropynyl butylcarbamate, benzalkonium chloride, 2 bromo-2-nitropropane-1,3-diol, 1,3-bis-(hydroxymethyl)-5,5-dimethylimidazolidine, benzisothiazolinone, 5-chloro-2-methylisothiazol-3(2H)-one, 2-methyl-isothiazol-3(2H)-one, cetyl pyridinium bromide and chloride, imidazolidinyl urea, diazolidinyl urea, poly(1-hexamethylene biguanidine hydrochloride)
and/or d) 1-90% by weight of one or more of the following sequestrants:
ethylenediamine tetraacetic acid and salts thereof, citric acid and salts thereof, ethylenediamine disuccinic acid and salts thereof, tetrasodium glutamate diacetate,
wherein the preservative composition optionally contains e) 0.1-50% by weight of one or more natural extracts, and the sum of a)-e) must equal 100% by weight.

5. A preservative composition according to any one of claims 1 to 4, comprising:
a) 5-80% by weight of a mixture of 4-10 well **characterised** fragrance raw materials with a cosmetic function, of which at least 4 are selected from:
allyl caproate, benzyl acetate, benzaldehyde, dihydroisojasmonate, ethyl phenethylacetal, ethyl cinnamate, ethyl methyl phenyl glycidate, ethyl vanillin, 2-heptylcyclopentanone, geranyl acetate, heliotropine, cis hex-3-en-1-ol, ethylene brassylate, nonalactone gamma, camphylcyclohexanol, undecalactone gamma, 2-t-butylcyclohexylacetate, pentyl salicylate, 2-phenylethanol and 2-phenylethyl acetate
and represent at least 40% by weight of the said mixture, and
b) 0-80% by weight of one or more of the following preservatives:
parahydroxy benzoic acid and salts thereof, (C₁-C₄) alkyl parahydroxy benzoates, sorbic acid and salts thereof, ortho hydroxybenzoic acid and salts thereof, benzoic acid and salts thereof, (C₁-C₂) alkyl benzoates, and
either c) 10-80% by weight of one or more of the following preservatives:
benzyl alcohol, 2-phenoxyethanol, cis 1-(3-chloroallyl)-3,5,7-triaza-1-azoniaadamantane chloride, iodopropynyl butylcarbamate, 2-bromo-2-nitropropane-1,3-diol, 1,3-bis-(hydroxymethyl)-5,5-dimethylimidazolidine, cetyl pyridinium bromide and chloride, imidazolidinyl urea, diazolidinyl urea, poly(1-hexamethylene biguanidine hydrochloride).
and/or d) 1-90% by weight of one or more of the following sequestrants:
ethylenediamine tetraacetic acid and salts thereof, citric acid and salts thereof, ethylenediamine disuccinic acid and salts thereof, tetrasodium glutamate diacetate,
wherein the preservative composition optionally contains e) 0.1-50% by weight of one or more natural extracts, and the sum of a)-e) must equal 100% by weight.

6. A preservative composition according to any one of claims 1 to 5 which comprises:
a) 5-25% by weight of a mixture of 2-10 well **characterised** fragrance raw materials with a cosmetic function, of which at least 2 materials are selected from:
allyl caproate, benzyl acetate, benzaldehyde, dihydroisojasmonate, ethyl phenethylacetal, ethyl cinnamate, ethyl methyl phenyl glycidate, ethyl vanillin, 2-heptylcyclopentanone, geranyl acetate, heliotropine, cis hex-3-en-1-ol, ethylene brassylate, nonalactone gamma, camphylcyclohexanol, undecalactone gamma, 2-t-butylcyclohexylacetate, pentyl salicylate, 2-phenylethanol and 2-phenylethyl acetate
and represent at least 20% by weight of the said mixture, and
b) 0-80% by weight of one or more of the following preservatives:
parahydroxy benzoic acid and salts thereof, (C₁-C₄) alkyl parahydroxy benzoates, sorbic acid and salts thereof, ortho hydroxybenzoic acid and salts thereof, benzoic acid and salts thereof, (C₁-C₂) alkyl benzoates, and
c) 10-80% by weight of one or more of the following preservatives:
benzyl alcohol, 2-phenoxyethanol, cis 1-(3-chloroallyl)-3,5,7-triaza-1-azoniaadamantane chloride, iodopropynyl butylcarbamate, 2-bromo-2-nitropropane-1,3-diol, 1,3-bis-(hydroxymethyl)-5,5-dimethylimidazolidine, cetyl pyridinium bromide and chloride, imidazolidinyl urea, diazolidinyl urea, poly(1-hexamethylene biguanidine hydrochloride).
and/or d) 1-90% by weight of one or more of the following sequestrants:
ethylenediamine tetraacetic acid and salts thereof, citric acid and salts thereof, ethylenediamine disuccinic acid and salts thereof, tetrasodium glutamate diacetate,
wherein the preservative composition optionally contains e) 0.1-50% by weight of one or more natural extracts, and the sum of a)-e) must equal 100% by weight.

7. A preservative composition according to any one of claims 1 to 6, which comprises:
a) 5-80% by weight of a mixture of 2-10 well **characterised** fragrance raw materials with a cosmetic function, of which at least 2 materials are selected from:
allyl caproate, benzyl acetate, benzaldehyde, dihydroisojasmonate, ethyl cinnamate, ethyl methyl phenyl glycidate, ethyl vanillin, geranyl acetate, heliotropine, cis hex-3-en-1-ol, ethylene brassylate, nonalactone gamma, camphylcyclohexanol, undecalactone gamma, pentyl salicylate, 2-t-butylcyclohexylacetate
and represent at least 20% by weight of the said mixture, and
b) 0-80% by weight of one or more of the following preservatives:
parahydroxy benzoic acid and salts thereof, (C₁-C₄) alkyl parahydroxy benzoates, sorbic acid and salts thereof, ortho hydroxybenzoic acid and salts thereof, benzoic acid and salts thereof, (C₁-C₂) alkyl benzoates, and
either c) 10-80% by weight of one or more of the following preservatives
benzyl alcohol, 2-phenoxyethanol, cis 1-(3-chloroallyl)-3,5,7-triaza-1-azoniaadamantane chloride, iodopropynyl butylcarbamate, 2-bromo-2-nitropropane-1,3-diol, 1,3-bis-(hydroxymethyl)-5,5-dimethylimidazolidine, cetyl pyridinium bromide and chloride, imidazolidinyl urea, diazolidinyl urea, poly(1-hexamethylene biguanidine hydrochloride).
and/or d) 1-90% by weight of one or more of the following sequestrants:
ethylenediamine tetraacetic acid and salts thereof, citric acid and salts thereof, ethylenediamine disuccinic acid and salts thereof, tetrasodium glutamate diacetate,
wherein the preservative composition optionally contains d) 0.1-50% by weight of one or more natural extracts, and the sum of a)-e) must equal 100% by weight.

8. A preservative composition according to claim 6 which comprises:
a) 5-80% by weight of a mixture of 2-10 well **characterised** fragrance raw materials with a cosmetic function, of which at least 4 materials are selected from:
allyl caproate, benzyl acetate, benzaldehyde, dihydroisojasmonate, ethyl cinnamate, ethyl methyl phenyl glycidate, ethyl vanillin, geranyl acetate, heliotropine, cis hex-3-en-1-ol, ethylene brassylate, nonalactone gamma, camphylcyclohexanol, undecalactone gamma, pentyl salicylate, 2-t-butylcyclohexylacetate
and represent at least 20% by weight of the said mixture, and
b) 0-80% by weight of one or more of the following preservatives:
(C₁-C₃) alkyl parahydroxy benzoates, sorbic acid and salts thereof, benzoic acid and salts thereof
and either c) 10-80% by weight of one or more of the following preservatives:
benzyl alcohol, 2-phenoxyethanol, cis 1-(3-chloroallyl)-3,5,7-triaza-1-azoniaadamantane chloride, iodopropynyl butylcarbamate, 2-bromo-2-nitropropane-1,3-diol, 1,3-bis-(hydroxymethyl)-5,5-dimethylimidazolidine, cetyl pyridinium bromide and chloride, imidazolidinyl urea, diazolidinyl urea, poly(1-hexamethylene biguanidine hydrochloride);
and/or d) 1-90% by weight of one or more of the following sequestrants:
ethylenediamine tetraacetic acid and salts thereof, citric acid and salts thereof, ethylenediamine disuccinic acid and salts thereof, tetrasodium glutamate diacetate,
wherein the preservative composition optionally contains e) 0.1-50% by weight of one or more natural extracts, and the sum of a)-e) must equal 100% by weight.

9. A preservative composition according to claim 7 or 8, which comprises:
a) 5-25% by weight of a mixture of 2-10 well **characterised** fragrance raw materials with a cosmetic function, of which at least 2 materials are selected from:
allyl caproate, benzyl acetate, benzaldehyde, dihydroisojasmonate, ethyl cinnamate, ethyl methyl phenyl glycidate, ethyl vanillin, geranyl acetate, heliotropine, cis hex-3-en-l-ol, ethylene brassylate, nonalactone gamma, camphylcyclohexanol, undecalactone gamma, pentyl salicylate, 2-t-butylcyclohexylacetate
and represent at least 20% by weight of the said mixture, and
b) 0-80% by weight of one or more of the following preservatives:
(C₁-C₃) alkyl parahydroxy benzoates, sorbic acid and salts thereof, benzoic acid and salts thereof and
either c) 10-80% by weight of one or more of the following preservatives:
benzyl alcohol, 2-phenoxyethanol, cis 1-(3-chloroallyl)-3,5,7-triaza-l-azoniaadamantane chloride, iodopropynyl butylcarbamate, 2-bromo-2-nitropropane-1,3-diol, 1,3-bis-(hydroxymethyl)-5,5-dimethylimidazolidine, cetyl pyridinium bromide and chloride, imidazolidinyl urea, diazolidinyl urea, poly(1-hexamethylene biguanidine hydrochloride);
and/or d) 1-90% by weight of one or more of the following sequestrants:
ethylenediamine tetraacetic acid and salts thereof, citric acid and salts thereof, ethylenediamine disuccinic acid and salts thereof, tetrasodium glutamate diacetate,
wherein the preservative composition optionally contains d) 0.1-50% by weight of one or more natural extracts, and the sum of a)-e) must equal 100% by weight.

10. A preservative composition according to any one of claims 7 to 9, which comprises:
a) 5-80% by weight of a mixture of 4-10 well **characterised** fragrance raw materials with a cosmetic function, of which at least 4 materials are selected from:
allyl caproate, benzyl acetate, benzaldehyde, dihydroisojasmonate, ethyl cinnamate, ethyl methyl phenyl glycidate, ethyl vanillin, geranyl acetate, heliotropine, cis hex-3-en-1-ol, ethylene brassylate, nonalactone gamma, camphylcyclohexanol, undecalactone gamma, pentyl salicylate, 2-t-butylcyclohexylacetate
and represent at least 40% by weight of the said mixture, and
b) 0-80% by weight of one or more of the following preservatives:
(C₁-C₃) alkyl parahydroxy benzoates, sorbic acid and salts thereof, benzoic acid and salts thereof and
either c) 10-80% by weight of one or more of the following preservatives:
benzyl alcohol, 2-phenoxyethanol, cis 1-(3-chloroallyl)-3,5,7-triaza-1-azoniaadamantane chloride, iodopropynyl butylcarbamate, 2-bromo-2-nitropropane-1,3-diol, 1,3-bis-(hydroxymethyl)-5,5-dimethylimidazolidine, cetyl pyridinium bromide and chloride, imidazolidinyl urea, diazolidinyl urea, poly(l-hexamethylene biguanidine hydrochloride);
and/or d) 1-90% by weight of one or more of the following sequestrants:
ethylenediamine tetraacetic acid and salts thereof, citric acid and salts thereof, ethylenediamine disuccinic acid and salts thereof, tetrasodium glutamate diacetate,
wherein the preservative composition optionally contains d) 0.1-50% by weight of one or more natural extracts, and the sum of a)-e) must equal 100% by weight.

11. Use of a preservative composition according to any one of claims 1 to 10 for improving the preservation of cosmetic, toiletry, personal care, household and laundry products containing at least 15% by weight of water and at least 1% by weight of an emulsifier or surfactant.

12. Use according to claim 11, in which the ethylene diamine tetracetic acid or its salts is present in said products as one of the sequestrants.

13. Use according to claim 10 or 11, in which the preservative c) contains phenoxyethanol.
